(19) Europäisches Patentamt
European Patent Office
Office européen des brevets

(11) **EP 3 461 903 A1**

(12) **DEMANDE DE BREVET EUROPEEN**

(43) Date de publication:
**03.04.2019   Bulletin 2019/14**

(51) Int Cl.:
*C12P 19/14* [(2006.01)]   *C12P 7/10* [(2006.01)]

(21) Numéro de dépôt: **18191589.3**

(22) Date de dépôt: **30.08.2018**

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**
Etats de validation désignés:
**KH MA MD TN**

(30) Priorité:  **28.09.2017   FR 1759032**

(71) Demandeur: **IFP Energies nouvelles
92852 Rueil-Malmaison Cedex (FR)**

(72) Inventeurs:
• **BATTISTA, Federico
73026 Melendugno (Lecce) (IT)**
• **GOMEZ ALMENDROS, Mélanie
69003 LYON (FR)**

(74) Mandataire: **IFP Energies nouvelles
Département Propriété Industrielle
Rond Point de l'échangeur de Solaize
BP3
69360 Solaize (FR)**

(54) **PROCÉDÉ D'HYDROLYSE ENZYMATIQUE A PARTIR D'UN MÉLANGE DE SUBSTRATS PRÉTRAITÉS DE POROSITES DIFFÉRENTES**

(57)    La présente invention concerne un procédé d'hydrolyse enzymatique dans lequel on met en contact, sous agitation, des substrats lignocellulosiques prétraités avec de l'eau et avec des enzymes de manière à ce que le mélange ait un taux de matière sèche entre 12 et 35% poids, ledit procédé étant caractérisé en ce qu'on utilise un mélange d'au moins deux substrats lignocellulosiques prétraités de porosités différentes, dont au moins un des substrats est un substrat dit de faible porosité possédant une porosité inférieure à 60 % volume et l'autre substrat est un substrat dit de haute porosité possédant une porosité supérieure ou égale à 60% volume, et ledit substrat de faible porosité est présent dans une quantité d'au moins 30% poids par rapport au poids total dudit mélange.

Figure 1

**EP 3 461 903 A1**

**Description**

## DOMAINE DE L'INVENTION

La présente invention concerne un procédé d'hydrolyse enzymatique à partir d'un mélange de substrats lignocellulosiques prétraités de porosités différentes permettant la transformation de la cellulose en glucose. Le glucose peut ensuite être utilisé dans diverses étapes ultérieures telles que par exemple dans une étape de fermentation pour la production d'alcools, ou pour la production d'intermédiaires pour la chimie.

## ART ANTÉRIEUR

La mise au point de procédés économiquement viables de valorisation de la biomasse lignocellulosique est aujourd'hui un vaste sujet d'actualité. La raréfaction des ressources fossiles et la concurrence avec les ressources alimentaires conduisent à chercher de nouvelles voies pour la production de biocarburants et d'intermédiaires chimiques.

Depuis les années 1970, la transformation de la biomasse lignocellulosique après hydrolyse des polysaccharides constitutifs en sucres a fait l'objet de nombreux travaux.

La biomasse lignocellulosique se caractérise par une structure complexe constituée de trois principaux polymères : la cellulose, les hémicelluloses et la lignine, dont la proportion varie suivant les espèces de biomasse lignocellulosique. Une composition typique mais non limitative est la suivante : la cellulose est présente à hauteur de 35 à 50%, les hémicelluloses qui sont des polysaccharides essentiellement constitués de pentoses et d'hexoses sont présents à hauteur de 20 à 30% et les lignines à hauteur de 15 à 25% poids. La dégradation de la biomasse se révèle difficile car les polysaccharides de la paroi végétale (cellulose et hémicelluloses) sont intimement associés à de la lignine qui confère aux parois leur rigidité.

De ces trois polymères, la cellulose est la principale source de sucres car elle est constituée de glucose, ce dernier pouvant être facilement valorisé.

De façon classique, les procédés de valorisation de la biomasse par voie biochimique comprennent plusieurs étapes. Une première étape est la collecte et le transport de la biomasse lignocellulosique dans un centre de transformation de la biomasse. La deuxième étape est le prétraitement ou préhydrolyse de la biomasse qui permet de rendre la cellulose accessible aux enzymes et ainsi de produire un substrat lignocellulosique prétraité. La troisième étape d'hydrolyse enzymatique permet, grâce à l'utilisation d'une solution d'enzymes cellulolytiques et hémicellulolytiques produites par des microorganismes, et appelée cocktail enzymatique, la transformation de la cellulose en glucose. Ce glucose peut être ensuite valorisé en produits intermédiaires par exemple en éthanol lors d'une quatrième étape de fermentation par, en général, la levure *Saccharomyces cerevisiae*, ou en mélange acétone, butanol, éthanol (ABE) par fermentation par la levure *Clostridium acetobutylicum*. Une cinquième étape de distillation permet ensuite de concentrer les molécules obtenues. Le glucose peut également être valorisé en biocarburants (hydrogène, méthane).

Une des étapes clés est ainsi l'hydrolyse enzymatique. Dans l'étape d'hydrolyse enzymatique, ledit substrat lignocellulosique prétraité doit être mélangé avec une solution liquide contenant les enzymes cellulolytiques et hémicellulolytiques. L'objectif étant d'obtenir une concentration élevée en sucres, l'étape d'hydrolyse enzymatique doit se faire à des concentrations élevées en substrat lignocellulosique prétraité, c'est-à-dire à haute teneur en matière sèche. Il a été évalué que le procédé est économiquement viable, lorsque une concentration minimale de sucres de 8% poids est produite lors de l'hydrolyse enzymatique, ce qui ramène à une teneur en matière sèche d'environ 15 % poids (McIntosh, S., Zhang, Z., Palmer, J., Wong, H., Doherty, W.O.S., Vancov, T., 2016. Pilot-scale cellulosic ethanol production using eucalyptus biomass pre-treated by dilute acid and steam explosion. Biofules, bioproducts and biorefining 10 (4), 346-358). Le travail à une teneur élevée de matière sèche permet également de réduire le volume du réacteur et, par conséquent, de réduire les coûts économiques et énergétiques du processus (Larsen, J., Ostergaard Petersen, M., Thirup, L., Wen Li, H., Krogh Iversen, F., 2008. The IBUS process of lignocellulosic bioethanol close to a commercial reality. Chem. Eng. Technol. 31, 765-722).

Cependant, le mélange intime du substrat lignocellulosique prétraité avec ladite solution liquide contenant les enzymes cellulolytiques et hémicellulolytiques s'avère difficile lorsque les teneurs en matière sèche sont élevées. En effet, le début de l'hydrolyse enzymatique à haute teneur en matière sèche notamment pose des problèmes de mélange et d'homogénéisation. Le milieu réactionnel est très pâteux et visqueux ce qui demande une agitation spécifique beaucoup plus complexe que celle nécessaire en fin d'hydrolyse où le mélange réactionnel est devenu plus liquide.

Les problèmes de viscosité corrélés à un taux élevé de matière sèche sont connus. Cara et al. (Cara, C., Moya, M., Ballesteros, I., Negro, M.J., González, A., Ruiz, E., 2007. Influence of solid loading on enzymatic hydrolysis of steam exploded or liquid hot water pretreated olive tree biomass. Process Biochemistry, 42, 1003-1009) et Battista et al. (Battista, F., Fino, D., Mancini, G., Ruggeri, B., 2016. Mixing in digesters used to treat high viscosity substrates: The case of olive oil production wastes. Journal of Environmental Chemical Engineering 4, 915-923) ont démontré que lorsque la teneur en matière sèche est élevée, la complexité des polymères lignocellulosiques provoque une augmentation de la viscosité du milieu réactionnel et par conséquent un mauvais mélange au sein du bioréacteur.

De plus, on a pu démontrer une relation entre la viscosité et la porosité de la biomasse lignocellulosique prétraitée. L'hydrolyse enzymatique à un taux élevé de matière sèche est très difficile lorsque le substrat lignocellulosique est caractérisé par un degré de porosité élevé (Lewandowska, M., Szymanka, K., Kordala, N., Dabrowska, A., Bednarski, W., Juszczuk, A., 2016. Evaluation of mucor indicus and Saccharomyces cerevisiae capability to ferment hydrolysates of rape straw and Miscanthus giganteus as affected by the pretreatment method. Bioresource Technology 212, 262-270). Une porosité élevée du substrat lignocellulosique prétraité provoque une imprégnation d'eau élevée. Une imprégnation d'eau plus élevée réduit la quantité de liquide dans le milieu réactionnel et provoque en conséquence une augmentation de la viscosité en raison d'une dispersion et homogénéisation du substrat plus faibles dans le réacteur.

Afin de pallier ce problème de viscosité lorsqu'on travaille à un taux de matière sèche élevé, la demanderesse a mis au point dans ses recherches un nouveau procédé d'hydrolyse enzymatique en utilisant un mélange d'au moins deux substrats lignocellulosiques de porosités différentes, l'un ayant une haute porosité, l'autre ayant une basse porosité, les deux substrats étant utilisés dans un certain ratio.

Plus particulièrement, la présente invention porte sur un procédé d'hydrolyse enzymatique dans lequel on met en contact, sous agitation, des substrats lignocellulosiques prétraités avec de l'eau et avec des enzymes de manière à ce que le mélange ait un taux de matière sèche entre 12 et 35% poids, ledit procédé étant caractérisé en ce qu'on utilise un mélange d'au moins deux substrats lignocellulosiques prétraités de porosités différentes, dont au moins un des substrats est un substrat dit de faible porosité possédant une porosité inférieure à 60 % volume et l'autre substrat est un substrat dit de haute porosité possédant une porosité supérieure ou égale à 60% volume, et ledit substrat de faible porosité est présent dans une quantité d'au moins 30% poids par rapport au poids total dudit mélange.

La demanderesse propose ainsi un procédé d'hydrolyse enzymatique à partir d'un mélange de substrats de porosités différentes. De cette façon, il est possible de travailler à une teneur élevée en matière sèche sans avoir les problèmes rhéologiques dus à l'imprégnation d'eau des substrats à haute porosité. En effet, comme indiqué précédemment, les propriétés physiques des substrats influencent la viscosité du milieu réactionnel. Les substrats de haute porosité peuvent assimiler plus d'eau du milieu réactionnel et provoquent ainsi l'augmentation de la viscosité. Au contraire, les substrats de porosité inférieure ne provoquent pas ces problèmes de viscosité. En mélangeant un substrat de haute porosité avec un substrat de faible porosité on peut donc s'attendre à une amélioration du mélange par la baisse de la viscosité.

Cependant, la demanderesse a remarqué que l'utilisation des deux substrats dans un certain ratio permet d'observer un effet synergique au niveau de la baisse de viscosité. En effet, l'effet d'imprégnation d'eau par des substrats de haute porosité est annulé par les substrats à faible porosité lorsque la concentration des substrats à faible porosité est d'au moins 30% poids dans ledit mélange.

Un avantage de la présente invention est de fournir un procédé d'hydrolyse enzymatique dans lequel, grâce à la réduction de la viscosité, le temps de mélange est réduit.

Un autre avantage de la présente invention est de fournir un procédé d'hydrolyse enzymatique dans lequel il est possible de travailler avec des volumes de réacteurs réduits.

De plus, un autre avantage de la présente invention est de fournir un procédé d'hydrolyse enzymatique dans lequel on observe une réduction de la consommation d'énergie.

En outre, la possibilité de travailler à teneur élevée en matière sèche avec des substrats souvent traités séparément, permet de réduire le nombre et le volume des réacteurs.

Un autre avantage de la présente invention est de fournir un procédé d'hydrolyse enzymatique permettant un suivi et une adaptation simple à l'évolution du milieu réactionnel ne nécessitant pas de mesures complexes.

Selon une variante, le substrat de faible porosité est présent dans une quantité comprise entre 30 et 50 % poids, et de préférence comprise entre 40 et 50 % poids par rapport au poids total dudit mélange.

Selon une variante, le substrat dit de faible porosité possède une porosité inférieure à 58% volume.

Selon une variante, le substrat dit de faible porosité possède une densité apparente supérieure à 680 kg/m3.

Selon une variante, le substrat dit de faible porosité est le miscanthus.

Selon une variante, le substrat dit de haute porosité possède une porosité supérieure à 65 % volume.

Selon une variante, le substrat dit de haute porosité possède une densité apparente comprise entre 530 et 680 kg/m$^3$.

Selon une variante, le substrat dit de haute porosité est la paille de blé.

Selon une variante, les substrats lignocellulosiques prétraitées sont mis en contact à un taux de matière sèche entre 18 et 24% poids.

Selon une variante, le procédé opère à une température comprise entre 40 et 60°C, à un pH compris entre 4 et 6, et à pression atmosphérique.

Selon une variante, ledit procédé est mis en oeuvre dans un réacteur à alimentation séquentielle au cours de laquelle aucun soutirage du contenu du réacteur n'est effectué.

Selon une autre variante, ledit procédé est mis en oeuvre dans un réacteur batch.

Selon une variante, ledit procédé est suivi d'une étape de fermentation en présence d'un microorganisme alcooligène.

Selon une autre variante, ledit procédé est réalisé en présence d'un microorganisme alcooligène selon un procédé de saccharification et de fermentation simultanées dit procédé SSF.

## DESCRIPTION DÉTAILLÉE DE L'INVENTION

La biomasse lignocellulosique prétraitée est obtenue à partir de bois (feuillus et résineux), brut ou traité, de sous-produits de l'agriculture tels que la paille, de fibres de plantes, de cultures forestières, de résidus de plantes alcooligènes, sucrières et céréalières, de résidus de l'industrie papetière, de biomasse marine (par exemple macroalgues cellulosiques) ou de produits de transformations de matériaux lignocellulosiques.

Les substrats lignocellulosiques utilisés dans le procédé de l'invention sont issus de la biomasse prétraitée dans des conditions permettant de déstructurer la lignocellulose en modifiant les propriétés physiques et physico-chimiques du matériau lignocellulosique. L'étape de prétraitement peut se faire selon tous types de prétraitement de biomasse lignocellulosique connus de l'homme du métier. Une étape de conditionnement au préalable, incluant par exemple un broyage ou un épierrage peut être aussi réalisée. L'étape de prétraitement peut être un traitement thermique, chimique, mécanique et/ou enzymatique ou une combinaison de ces traitements.

Selon une variante préférée, l'étape de prétraitement est choisie parmi un prétraitement en conditions acides tels qu'une cuisson acide ou l'explosion à la vapeur en conditions acides, un prétraitement en milieux alcalins tels qu'un prétraitement au sulfure de sodium (procédé Kraft), un procédé ARP (selon la terminologie anglo-saxonne Ammonia Recycle Percolation) ou un procédé AFEX (selon la terminologie anglo-saxonne Ammonia Fiber Explosion), un prétraitement oxydant tels qu'un prétraitement utilisant l'ozone, le peroxyde d'hydrogène, l'oxygène ou l'acide peracétique, un prétraitement sans ajout de réactifs chimiques tel que l'explosion à la vapeur sans ajout d'acide ou le prétraitement par lavage à l'eau très chaude, ou encore un procédé organosolv.

Avantageusement, l'étape de prétraitement est un prétraitement par l'explosion à la vapeur en conditions acides, de préférence dans les conditions optimales, de 150 à 250 °C pendant quelques minutes.

Le mélange d'au moins deux substrats lignocellulosiques prétraités de porosités différentes utilisé dans le procédé selon l'invention comporte au moins un substrat dit de faible porosité possédant une porosité inférieure à 60 % volume et un autre substrat dit de haut porosité possédant une porosité supérieure ou égale à 60% volume. Le taux de porosité est mesuré par la méthode des isothermes d'adsorption-désorption d'azote (Horvath, G., Kawazoe, K., 1983. Method for calculation of effective pore size distribution in molecular sieve carbon, J. Chem. Eng. Jpn. 16, 470).

Le substrat dit de faible porosité possède une porosité inférieure à 60 % volume, et de préférence inférieure à 58% volume. Généralement, le substrat dit de faible porosité possède une porosité comprise entre 45 et inférieure à 60 % volume, et de préférence entre 48 et 58 % volume.

Le substrat dit de faible porosité se caractérise généralement par une densité apparente supérieure à 680 kg/m$^3$. La densité apparente est mesurée par le principe d'Archimède.

Le substrat dit de faible porosité est avantageusement le miscanthus. Le miscanthus a généralement une porosité comprise entre 49 et 55 % volume. Le miscanthus a généralement une densité apparente comprise entre 700 et 750 kg/m$^3$.

Le substrat dit de haute porosité possède une porosité supérieure ou égale à 60 % volume, de préférence supérieure à 65% volume. Généralement, le substrat dit de haute porosité possède une porosité comprise entre 60 et 80 % volume, de préférence entre 65 et 79 % volume.

Le substrat dit de haute porosité se caractérise généralement par une densité apparente comprise entre 530 et 680 kg/m$^3$, et de préférence entre 550 et 670 kg/m$^3$.

Le substrat dit de haute porosité est avantageusement la paille de blé. La paille de blé a généralement une porosité comprise entre 69 et 77% volume. La paille de blé a généralement une densité apparente comprise entre 570 et 650 kg/m$^3$.

Ledit substrat de faible porosité est présent dans une quantité d'au moins 30% poids, de préférence d'au moins 40 % poids, par rapport au poids total dudit mélange.

Ledit substrat de faible porosité est de préférence présent dans une quantité d'au plus 50% poids par rapport au poids total dudit mélange.

De préférence, le substrat de faible porosité est présent dans une quantité comprise entre 30 et 50 % poids, de préférence comprise entre 40 et 50 % poids, par rapport au poids total dudit mélange.

Dans toute la suite du texte, on exprime la concentration en substrat lignocellulosique prétraité en pourcentage poids de matière sèche. Le taux de matière sèche est mesuré selon la norme ASTM E1756-08(2015) « Standard Test Method for Determinatoin of Total Solids in Biomass ».

Conformément à l'invention, les substrats lignocellulosiques prétraités sont mis en contact dans le procédé selon la présente invention avec de l'eau et avec des enzymes de manière à ce que le mélange ait un taux de matière sèche entre 12 et 35% poids, de préférence entre 15 et 30% poids, et de manière préférée entre 18 et 24% poids.

Conformément à l'invention, les enzymes sont mises en contact dans le procédé selon la présente invention à une concentration comprise entre 0,1 à 60 mg d'enzymes par grammes de cellulose, de préférence à une concentration comprise entre 5 et 30 mg d'enzymes par grammes de cellulose et de manière préférée comprise entre 10 et 20 mg d'enzymes par grammes de cellulose.

L'hydrolyse enzymatique est généralement réalisée à un pH compris entre 4 et 6, de préférence compris entre 4,5 et 5,8 et encore plus préférentiellement entre 4,8 et 5,5. Elle se déroule généralement à une température entre 40 et 60 °C, et de préférence entre 50 et 55°C. Elle opère avantageusement à pression atmosphérique.

L'hydrolyse enzymatique est réalisée au moyen d'enzymes produites par un microorganisme. La solution enzymatique ajoutée contient des enzymes qui décomposent la cellulose en sucres. Des micro-organismes, comme les champignons appartenant aux genres *Trichoderma, Aspergillus, Penicillium ou Schizophyllum*, ou les bactéries anaérobies appartenant par exemple au genre *Clostridium*, produisent ces enzymes, contenant notamment les cellulases adaptées à l'hydrolyse poussée de la cellulose. De façon très préférée, les enzymes cellulolytiques de l'étape d) sont produits par le microorganisme *Trichoderma reesei*. Conformément à l'invention, la durée de mise en contact lors de l'hydrolyse enzymatique est comprise entre 1 et 200 heures, de préférence entre 2 et 120 heures et de manière préférée entre 24 et 120 heures.

Le procédé selon l'invention peut être effectué en mode continu ou discontinu (aussi appelé mode batch selon la terminologie anglo-saxonne), ou en alimentation séquentielle (aussi appelé mode fed-batch selon la terminologie anglo-saxonne), dans un ou plusieurs réacteurs. Selon une variante, le procédé selon l'invention est effectué de manière discontinue dans un réacteur fermé, aussi appelé un réacteur batch.

Ledit procédé selon la présente invention peut être suivi en mesurant au cours du temps la valeur de l'une des caractéristiques rhéologiques du milieu réactionnel qui sont avantageusement choisies parmi la viscosité du milieu réactionnel, le couple de torsion de l'arbre du système d'agitation et la puissance électrique consommée par le moteur. La puissance électrique consommée par le moteur est notée $P_{elec}$.

Durant le procédé selon l'invention, la viscosité du milieu réactionnel, le couple de torsion de l'arbre du système d'agitation et la puissance électrique consommée par le moteur sont des caractéristiques rhéologiques de suivi du substrat lignocellulosique produit qui présentent plusieurs intérêts. En effet, lesdites caractéristiques, viscosité, couple et puissance, sont reliées entre elles. La puissance électrique consommée par le moteur $P_{elec}$ est liée à la puissance mécanique $P_{méca}$ entrainant l'arbre d'agitation.

La puissance électrique consommée par le moteur est un paramètre classiquement mesuré et suivi sur les installations pilote ou industrielle.

Les formules suivantes définissent les relations entre les différents paramètres :

$P_{méca}$ = f ($P_{elec}$), f étant une caractéristique de conception du moteur et étant donnée par le constructeur du moteur.

$P_{méca} = 2\pi N*C$ dans laquelle :

N est la vitesse d'agitation en tour par seconde,

C est le couple en N.m,

et $P_{méca}$ est la puissance en watt.

En agitation on a la relation suivante :

$$P_{méca} = \rho N_p N^3 D^5$$

$\rho$ est la densité du milieu réactionnel en $kg.m^{-3}$

D est le diamètre de l'agitateur en m,

$N_p$ est une caractéristique de l'agitateur dépendant de la géométrie de la cuve et du régime d'écoulement.

En régime d'écoulement laminaire, on a la relation suivante :

$$N_p = A/Re \text{ d'où } P_{méca} = \rho A N^3 D^5/Re$$

avec A étant une constante du système d'agitation et Re le nombre de Reynolds et

$$Re = \rho N D^2 / \overline{\mu},$$

$\overline{\mu}$ étant la viscosité dynamique moyenne mesurée en Pascal seconde (Pa.s) du milieu réactionnel avec $\overline{\mu}= P_{méca}/(AN^2D^3)$

$= 2\pi C/(AD^3N)$

Si la viscosité et le couple de torsion de l'arbre du système d'agitation sont des mesures facilement accessibles à petite échelle, la puissance électrique consommée par le moteur $P_{elec}$ est la grandeur la plus facilement mesurable à échelle industrielle.

De manière très préférée, ledit procédé selon la présente invention se caractérise en ce qu'on réalise une mesure au cours du temps de la puissance électrique consommée par le moteur.

Ledit procédé selon la présente invention est avantageusement réalisé dans un réacteur, de préférence de forme cylindrique, présentant un ratio hauteur/diamètre avantageusement compris entre 1 et 3.

Ledit réacteur permet de traiter des milieux visqueux présentant une viscosité variable et ainsi de mettre en oeuvre des taux de matière sèche en substrat lignocellulosique pouvant atteindre les 35 % poids. Les taux de matière sèche élevés et la viscosité importante du milieu réactionnel requièrent que le réacteur soit équipé d'un agitateur permettant un bon contact entre enzymes et substrat et une bonne homogénéité. Classiquement, l'agitateur choisi doit pouvoir traiter des écoulements laminaires. Des agitateurs larges, voire raclant la paroi du réacteur avec des vitesses de rotation modérée et exerçant une action de malaxage et pétrissage sont préférés. Un exemple d'agitateur particulièrement adapté est le Paravisc® (EKATO) qui permet aussi l'ajout d'une contre-pâle cassant les mouvements d'ensemble.

La vitesse d'agitation dépend de la taille du réacteur et de l'agitateur.

Dans le cas où l'on mesure au cours du temps la valeur de la puissance électrique consommée par le moteur, ladite puissance électrique consommée par le moteur ramenée à la masse du volume réactionnel reste avantageusement comprise entre 0,05 et 4kW/tonne et de préférence entre 0,5 et 2kW/tonne.

Selon un mode de réalisation préféré, le procédé d'hydrolyse enzymatique selon l'invention peut être suivi d'une étape de fermentation alcoolique par un microorganisme alcooligène de manière à produire un effluent fermenté contenant de l'alcool.

L'hydrolyse enzymatique et la fermentation alcoolique peuvent aussi être opérées de manière simultanée. Dans ce cas on parle d'un procédé "SSF" (selon le terme anglo-saxon pour Simultaneous Saccharification and Fermentation). L'hydrolyse enzymatique et la fermentation alcoolique peuvent aussi être mises en oeuvre selon d'autres agencements connus de l'homme du métier, tel que le procédé "PSSF" (Presaccharification followed by Simultaneous Saccharification and Fermentation selon le terme anglo-saxon) ou encore le procédé "HHF" (Hybrid Hydrolysis and Fermentation selon le terme anglo-saxon).

Les sucres obtenus par hydrolyse enzymatique peuvent être fermentés en alcools tel que l'éthanol, le 1,3-propanediol, l'isopropanol, le 1-butanol, l'isobutanol ou le 1,4-butanediol, seul ou en mélange. De préférence, la fermentation alcoolique produit de l'éthanol.

La fermentation alcoolique est assurée par des levures ou autres microorganismes alcooligènes. Au sens de la présente invention, le terme "fermentation alcoolique" désigne un procédé de fermentation des sucres en alcool(s) au seul moyen de microorganismes. Les microorganismes alcooligènes utilisés pendant l'étape de fermentation alcoolique des hexoses sont de préférence choisis parmi les levures et les bactéries, éventuellement génétiquement modifiées.

Lorsque le microorganisme alcooligène est une levure, *Saccharomyces cerevisiae* est celle qui est la plus performante. Il est également possible de choisir des levures telles que *Schizosaccharomyces pombe* ou *Saccharomyces uvarum* ou *diastaticus.* Des levures plus thermophiles, telles que les *Kluyveromyces fragilis* (maintenant souvent désignée par *K. marxianus*) présentent également un intérêt, notamment lorsque l'hydrolyse enzymatique et la fermentation alcoolique sont réalisées simultanément (procédé SSF).

Un organisme génétiquement modifié, comme par exemple une levure de type *Saccharomyces cerevisiae* telle que la TMB 3400 (Ohgren et al, J. of Biotech 126, 488-498, 2006) peut également être utilisé.

Lorsque le microorganisme alcooligène est une bactérie, on préférera *Zymomonas mobilis* qui présente une voie d'assimilation efficace pour la production d'éthanol, ou les bactéries anaérobies du genre *Clostridium,* comme par exemple, *Clostridium acetobutylicum* pour la production de mélanges d'alcools et solvants comme acétone-butanol-éthanol (ABE) ou isopropanol-butanol-éthanol (IBE), ou encore *Escherichia coli* pour la production d'isobutanol par exemple.

La fermentation alcoolique est réalisée préférentiellement à une température comprise entre 30°C et 40°C, et un pH entre 3 et 6,5.

Les levures, et de préférence *Saccharomyces cerevisiae* sont les microorganismes utilisés de façon très préférée. Ils présentent une meilleure robustesse, sécurité, et ne nécessitent pas de stérilité pour la conduite du procédé et des installations.

Les levures du genre *Saccharomyces* sont capables de fermenter les seuls et uniques hexoses (glucose et mannose essentiellement). Ces levures valorisent de façon optimale les hexoses en éthanol et permettent d'atteindre de bons rendements de conversion.

Lorsque l'hydrolyse enzymatique et la fermentation alcoolique sont réalisées dans une même et seule opération (procédé SSF), de préférence la température est comprise entre 30 et 45°C, et le pH compris entre 4 et 6 afin de favoriser la performance des levures.

L'exemple de fonctionnement ci-après permet d'illustrer le procédé selon l'invention.

## EXEMPLES

Les substrats utilisés pour les exemples de tests sont respectivement la paille de blé et le miscanthus prétraités. Le tableau 1 résume les caractéristiques physiques et chimiques de la paille de blé et du miscanthus prétraités. Les substrats prétraités présentent généralement une matière sèche comprise entre 40 et 60 % poids, ladite matière sèche du tableau se référant au substrat prétraité en tant que tel. Des essais batch sur un substrat de haute porosité (paille de blé), sur un substrat à faible porosité (miscanthus) et sur une combinaison des deux à différents ratios ont été conduits (tableau 2). Tous les tests ont été réalisés à un taux de matière sèche de 20 % poids dans le mélange du procédé.

Les performances des tests ont été évaluées en tenant compte des trois facteurs: (i) le temps de mélange, (ii) la consommation énergétique du mélange et (iii) la concentration de glucose contenu dans le mélange réactionnel à la fin de l'hydrolyse enzymatique.

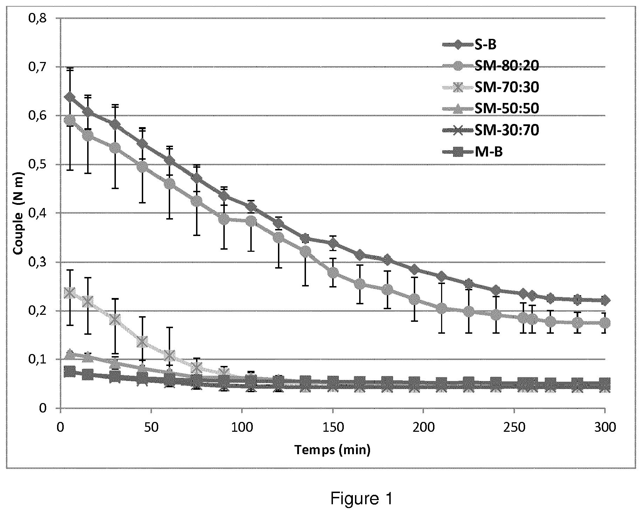

Les résultats montrent que le comportement rhéologique lors de l'hydrolyse enzymatique dépend des substrats utilisés pour alimenter le réacteur (Figure 1): le couple est plus élevé pour la paille de blé que pour le miscanthus. La paille de blé (essai S-B) a enregistré une diminution considérable des valeurs de couple entre le début et la fin des essais. En fait, le couple requis pour un bon mélange du milieu réactionnel passe de 0,64 Nm à 0,22 Nm pour le test S-B. D'autre part, le couple dans le cas du test miscanthus (essai M-B) passe de 0,09 à 0,045 Nm (figure 1).

La valeur du couple est strictement corrélée à la consommation énergétique pour le mélange. Par conséquent, l'essai S-B nécessitait plus de 32,5 kJ, par contre l'essai M-B nécessitait entre 5 et 7 kJ, avec une réduction énergétique de près de 80%. Le temps de mélange, qui est un indicateur des performances rhéologiques dans le réacteur, était plus de 50 s pour la paille de blé contre seulement 17 s pour le miscanthus.

La concentration en glucose était semblable pour tous les essais : entre 19 et 21 g/L.

Les différentes valeurs rhéologiques et énergétiques pour les deux substrats peuvent être expliquées par la viscosité. Le milieu réactionnel composé de 20% poids de matière sèche de paille de blé (essai S-B) est très visqueux, ayant une viscosité apparente de 420,1 $\pm$ 3,1 cP. Au contraire, le miscanthus (essai M-B) présente un comportement fluide-dynamique doux avec des valeurs de viscosité faible 79,40 $\pm$ 4,05 cP à 20% poids de matière sèche. Cette différence est due à une structure physique différente des deux substrats. Le tableau 1 montre que la paille de blé a un degré de porosité plus élevé que le miscanthus (73 % volume et 52 % volume respectivement). Le comportement rhéologique peut alors s'expliquer par une imprégnation d'eau bien supérieure pour les particules avec une porosité plus élevée (paille de blé).

De plus, des essais batch (tableau 2) ont été effectués sur des mélanges de paille de blé et de miscanthus à différentes concentrations. Comme attendu, la diminution de la concentration de paille de blé dans le mélange a permis de diminuer les temps de mélange et les consommations énergétiques. Dans l'essai SM-80:20, la viscosité était encore très influencée par la présence de paille de blé. Cependant, lorsque le taux de miscanthus présent dans le mélange réactionnel atteint 30% poids (essai SM-70:30), la viscosité du mélange baisse et son comportement est semblable à celui observé lors du test M-B.

La réduction des valeurs de couple par l'augmentation de la teneur en miscanthus dans le mélange réactionnel a une conséquence importante sur la consommation énergétique. L'essai SM-80:20 requis pour le mélange de 30,5 kJ, semblable au test S-B, mais la consommation énergétique tombe à près de 8 kJ pour l'essai SM-70:30. C'est une valeur très proche des 5,8 kJ enregistrés avec l'essai M-B. On observe donc un effet synergique au niveau de la baisse de viscosité. En effet, l'effet d'imprégnation d'eau par des substrats de haute porosité est annulé par les substrats à faible porosité lorsque la concentration des substrats à faible porosité est d'au moins 30% poids dans ledit mélange.

Tableau 1. Description de la paille de blé et de miscanthus

|  | Miscanthus | Paille de blé |
| --- | --- | --- |
| Matière Sèche du substrat prétraité (% poids) | 47,73 $\pm$ 1,44 | 46,06 $\pm$ 1,90 |
| Densité apparente (kg/m$^3$) | 722,54 $\pm$ 12,14 | 607,46 $\pm$ 18,76 |
| Porosité (% volume) | 52,00 $\pm$ 2,60 | 73,00 $\pm$ 3,65 |

Tableau 2. Abréviation et description des tests.

| Abréviation | Description des tests |
|---|---|
| S-B | Batch test avec paille de blè |
| M-B | Batch test avec miscanthus |
| SM-80:20 | Batch test un mélange composé par 80 % poids de paille de blé et 20 % poids de miscanthus |
| SM-70:30 | Batch test un mélange composé par 70 % poids de paille de blé et 30 % poids de miscanthus |
| SM-50:50 | Batch test un mélange composé par 50 % poids de paille de blé et 50 % poids de miscanthus |
| SM-30:70 | Batch test un mélange composé par 30 % poids de paille de blé et 70 % poids de miscanthus |

**Revendications**

1. Procédé d'hydrolyse enzymatique dans lequel on met en contact, sous agitation, des substrats lignocellulosiques prétraités avec de l'eau et avec des enzymes de manière à ce que le mélange ait un taux de matière sèche entre 12 et 35% poids, ledit procédé étant **caractérisé en ce qu'**on utilise un mélange d'au moins deux substrats lignocellulosiques prétraités de porosités différentes, dont au moins un des substrats est un substrat dit de faible porosité possédant une porosité inférieure à 60 % volume et l'autre substrat est un substrat dit de haute porosité possédant une porosité supérieure ou égale à 60% volume, et ledit substrat de faible porosité est présent dans une quantité d'au moins 30% poids par rapport au poids total dudit mélange.

2. Procédé selon la revendication 1 dans lequel le substrat de faible porosité est présent dans une quantité comprise entre 30 et 50 % poids par rapport au poids total dudit mélange.

3. Procédé selon la revendication 1 ou 2 dans lequel le substrat de faible porosité est présent dans une quantité comprise entre 40 et 50 % poids par rapport au poids total dudit mélange.

4. Procédé selon l'une des revendications 1 à 3 dans lequel le substrat dit de faible porosité possède une porosité inférieure à 58% volume.

5. Procédé selon l'une des revendications 1 à 4 dans lequel le substrat dit de faible porosité possède une densité apparente supérieure à 680 kg/m3.

6. Procédé selon l'une des revendications 1 à 5 dans lequel le substrat dit de faible porosité est le miscanthus.

7. Procédé selon l'une des revendications 1 à 6 dans lequel le substrat dit de haute porosité possède une porosité supérieure à 65 % volume.

8. Procédé selon l'une des revendications 1 à 7 dans lequel le substrat dit de haute porosité possède une densité apparente comprise entre 530 et 680 kg/m$^3$.

9. Procédé selon l'une des revendications 1 à 8 dans lequel le substrat dit de haute porosité est la paille de blé.

10. Procédé selon l'une des revendications 1 à 9 dans lequel les substrats lignocellulosiques prétraitées sont mis en contact à un taux de matière sèche entre 18 et 24% poids.

11. Procédé selon l'une des revendications 1 à 10 dans lequel le procédé opère à une température comprise entre 40 et 60°C, à un pH compris entre 4 et 6, et à pression atmosphérique.

12. Procédé selon l'une des revendications 1 à 11 dans lequel ledit procédé est mis en oeuvre dans un réacteur à alimentation séquentielle au cours de laquelle aucun soutirage du contenu du réacteur n'est effectué.

13. Procédé selon l'une des revendications 1 à 11 dans lequel ledit procédé est mis en oeuvre dans un réacteur batch.

14. Procédé selon l'une des revendications 1 à 13 dans lequel ledit procédé est suivi d'une étape de fermentation en présence d'un microorganisme alcooligène.

**15.** Procédé selon l'une des revendications 1 à 13 dans lequel ledit procédé est réalisée en présence d'un microorganisme alcooligène selon un procédé de saccharification et de fermentation simultanées.

Figure 1

Europäisches Patentamt

European Patent Office

Office européen des brevets

# RAPPORT DE RECHERCHE EUROPEENNE

Numéro de la demande

EP 18 19 1589

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | RONDINELE DE OLIVEIRA MOUTTA ET AL: "Comparative Response and Structural Characterization of Sugarcane Bagasse, Straw and Bagasse-Straw 1:1 Mixtures Subjected to Hydrothermal Pretreatment and Enzymatic Conversion", JOURNAL OF MICROBIAL & BIOCHEMICAL TECHNOLOGY, vol. 01, no. S12, 1 janvier 2013 (2013-01-01), pages 1-8, XP055480800, DOI: 10.4172/1948-5948.S12-005 * abrégé * * page 2, colonne de gauche, alinéa 1 - colonne de droite, alinéa 5 * * page 3, colonne de gauche, alinéa 5 - page 4, colonne de droite, alinéa 1; figure 2 * | 1-15 | INV. C12P19/14 C12P7/10 |
| | ----- | | |
| A | Glauber Cruz ET AL: "INVESTIGATION OF POROSITY, WETTABILITY AND MORPHOLOGY OF THE CHEMICALLY PRETREATED SUGARCANE BAGASSE", 22nd International Congress of Mechanical Engineering November 3-7, 2013, Ribeirão Preto, SP, Brazil, 1 novembre 2013 (2013-11-01), pages 1-13, XP055480830, Extrait de l'Internet: URL:https://www.researchgate.net/publicati on/261872294_INVESTIGATION_OF_POROSITY_WET TABILITY_AND_MORPHOLOGY_OF_THE_CHEMICALLY_ PRETREATED_SUGARCANE_BAGASSE [extrait le 2018-06-04] * page 2, alinéa 3 * * page 3, alinéa 4 - page 6, alinéa 2; tableau 1 * * page 9, alinéa 5 - page 11, alinéa 2 * | 1-15 | DOMAINES TECHNIQUES RECHERCHES (IPC) C12P |
| | ----- | | |
| | -/-- | | |

Le présent rapport a été établi pour toutes les revendications

| Lieu de la recherche | Date d'achèvement de la recherche | Examinateur |
|---|---|---|
| Berlin | 13 février 2019 | Mateo Rosell, A |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

page 1 de 4

Europäisches
Patentamt

European
Patent Office

Office européen
des brevets

**RAPPORT DE RECHERCHE EUROPEENNE**

Numéro de la demande

EP 18 19 1589

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | MODENBACH ALICIA A ET AL: "Enzymatic hydrolysis of biomass at high-solids loadings - A review", BIOMASS AND BIOENERGY, vol. 56, septembre 2013 (2013-09), pages 526-544, XP028684730, ISSN: 0961-9534, DOI: 10.1016/J.BIOMBIOE.2013.05.031 * page 526, colonne de gauche, alinéa 1 - page 530, colonne de gauche, alinéa 1 * * page 532, colonne de gauche, alinéa 1 - page 535, colonne de droite, alinéa 1 * ----- | 1-15 | |
| A | Yu Zhang ET AL: "HIGH SOLID AND LOW ENZYME LOADING BASED SACCHARIFICATION OF AGRICULTURAL BIOMASS", BioResources, 7(1), 1 janvier 2012 (2012-01-01), pages 345-353, XP055480999, Extrait de l'Internet: URL:http://ojs.cnr.ncsu.edu/index.php/BioR es/article/view/BioRes_07_1_0345_Zhang_LXY Z_Hi_Solid_Lo_Enzyme_Saccharification_Ag/1 340 [extrait le 2018-06-04] * abrégé * * page 348, alinéa 1 - page 350, dernier alinéa; figure 3 * ----- -/-- | 1-15 | |

DOMAINES TECHNIQUES RECHERCHES (IPC)

Le présent rapport a été établi pour toutes les revendications

| Lieu de la recherche | Date d'achèvement de la recherche | Examinateur |
|---|---|---|
| Berlin | 13 février 2019 | Mateo Rosell, A |

CATEGORIE DES DOCUMENTS CITES

X : particulièrement pertinent à lui seul
Y : particulièrement pertinent en combinaison avec un
autre document de la même catégorie
A : arrière-plan technologique
O : divulgation non-écrite
P : document intercalaire

T : théorie ou principe à la base de l'invention
E : document de brevet antérieur, mais publié à la
date de dépôt ou après cette date
D : cité dans la demande
L : cité pour d'autres raisons
......................................................................
& : membre de la même famille, document correspondant

EPO FORM 1503 03.82 (P04C02)

Europäisches
Patentamt

European
Patent Office

Office européen
des brevets

**RAPPORT DE RECHERCHE EUROPEENNE**

Numéro de la demande

EP 18 19 1589

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | CLAUDIA I. ISHIZAWA ET AL: "Porosity and Its Effect on the Digestibility of Dilute Sulfuric Acid Pretreated Corn Stover", JOURNAL OF AGRICULTURAL AND FOOD CHEMISTRY, vol. 55, no. 7, 1 avril 2007 (2007-04-01), pages 2575-2581, XP055480685, US ISSN: 0021-8561, DOI: 10.1021/jf062131a * abrégé * * page 2577, colonne de gauche, alinéa 6 - page 2579, colonne de gauche, alinéa 3; figures 1-5; tableau 3 * | 1-15 | |
| A | MAGNUS WIMAN ET AL: "Cellulose accessibility determines the rate of enzymatic hydrolysis of steam-pretreated spruce", BIORESOURCE TECHNOLOGY, vol. 126, 1 décembre 2012 (2012-12-01), pages 208-215, XP055481030, AMSTERDAM, NL ISSN: 0960-8524, DOI: 10.1016/j.biortech.2012.08.082 * abrégé * * page 212, colonne de droite, alinéa 2 - page 215, colonne de gauche, dernier alinéa; tableaux 2,3 * | 1-15 | **DOMAINES TECHNIQUES RECHERCHES (IPC)** |

-/--

Le présent rapport a été établi pour toutes les revendications

| Lieu de la recherche | Date d'achèvement de la recherche | Examinateur |
|---|---|---|
| Berlin | 13 février 2019 | Mateo Rosell, A |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

page 3 de 4

Europäisches
Patentamt

European
Patent Office

Office européen
des brevets

**RAPPORT DE RECHERCHE EUROPEENNE**

Numéro de la demande

EP 18 19 1589

| | DOCUMENTS CONSIDERES COMME PERTINENTS | | | |
|---|---|---|---|
| Catégorie | Citation du document avec indication, en cas de besoin, des parties pertinentes | Revendication concernée | CLASSEMENT DE LA DEMANDE (IPC) |
| T | FEDERICO BATTISTA ET AL: "Enzymatic hydrolysis at high dry matter content: The influence of the substrates' physical properties and of loading strategies on mixing and energetic consumption", BIORESOURCE TECHNOLOGY, vol. 250, 20 novembre 2017 (2017-11-20), - 1 février 2018 (2018-02-01), pages 191-196, XP055480461, AMSTERDAM, NL ISSN: 0960-8524, DOI: 10.1016/j.biortech.2017.11.049 * le document en entier * ----- | | |

DOMAINES TECHNIQUES
RECHERCHES (IPC)

Le présent rapport a été établi pour toutes les revendications

| Lieu de la recherche | Date d'achèvement de la recherche | Examinateur |
|---|---|---|
| Berlin | 13 février 2019 | Mateo Rosell, A |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.................................................................
& : membre de la même famille, document correspondant

EPO FORM 1503 03.82 (P04C02)

page 4 de 4

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **MCLNTOSH, S. ; ZHANG, Z. ; PALMER, J. ; WONG, H. ; DOHERTY, W.O.S. ; VANCOV, T.** Pilot-scale cellulosic ethanol production using eucalyptus biomass pre-treated by dilute acid and steam explosion. *Biofules, bioproducts and biorefining,* 2016, vol. 10 (4), 346-358
- **LARSEN, J. ; OSTERGAARD PETERSEN, M. ; THIRUP, L. ; WEN LI, H. ; KROGH IVERSEN, F.** The IBUS process of lignocellulosic bioethanol close to a commercial reality. *Chem. Eng. Technol.,* 2008, vol. 31, 765-722
- **CARA, C. ; MOYA, M. ; BALLESTEROS, I. ; NEGRO, M.J. ; GONZÁLEZ, A. ; RUIZ, E.** Influence of solid loading on enzymatic hydrolysis of steam exploded or liquid hot water pretreated olive tree biomass. *Process Biochemistry,* 2007, vol. 42, 1003-1009

- **BATTISTA, F. ; FINO, D. ; MANCINI, G. ; RUGGERI, B.** Mixing in digesters used to treat high viscosity substrates: The case of olive oil production wastes. *Journal of Environmental Chemical Engineering,* 2016, vol. 4, 915-923
- **LEWANDOWSKA, M. ; SZYMANKA, K. ; KORDALA, N. ; DABROWSKA, A. ; BEDNARSKI, W. ; JUSZCZUK, A.** Evaluation of mucor indicus and Saccharomyces cerevisiae capability to ferment hydrolysates of rape straw and Miscanthus giganteus as affected by the pretreatment method. *Bioresource Technology,* 2016, vol. 212, 262-270
- **HORVATH, G. ; KAWAZOE, K.** Method for calculation of effective pore size distribution in molecular sieve carbon. *J. Chem. Eng. Jpn.,* 1983, vol. 16, 470
- **OHGREN et al.** *J. of Biotech,* 2006, vol. 126, 488-498